# EUROPÄISCHE PATENTSCHRIFT

(11) **EP 1 355 896 B1**
(45) Veröffentlichungstag und Bekanntmachung des Hinweises auf die Patenterteilung: **12.01.2005**
(21) Anmeldenummer: 02718036.3
(22) Anmeldetag: 23.01.2002
(51) Int. Cl.: C07D 303/42

(54) **VERFAHREN ZUR HERSTELLUNG VON EPOXIDIERTEN GLYCERIDACETATEN**
METHOD FOR PRODUCING EPOXIDATED GLYCERIDE ACETATES
PROCEDE DE PRODUCTION D'ACETATES DE GLYCERIDE EPOXYDES

(30) Priorität: 01.02.2001 DE 10104815
(43) Veröffentlichungstag der Anmeldung: 29.10.2003
(73) Patentinhaber: Cognis Deutschland GmbH & Co. KG, 40589 Düsseldorf (DE)
(72) Erfinder: DAUTE, Peter, 27616 Beverstadt (DE); PICARD, Ralf, 27572 Bremerhaven (DE); KLAMANN, Jörg-Dieter, 4051 Alderley, Queensland (AU); WEDL, Peter, 27568 Bremerhaven (DE); PETERS, Artur, 27612 Loxstedt (DE)
(86) Internationale Anmeldenummer: PCT/EP2002/000618
(87) Internationale Veröffentlichungsnummer: WO 2002/060884

(56) Entgegenhaltungen:
- WO-A-90/12858
- US-A- 2 895 966
- US-A- 3 049 504

## Beschreibung

### Gebiet der Erfindung

Die Erfindung betrifft ein Verfahren zur Herstellung von epoxidierten Glyceridacetaten. Dabei werden Epoxyfettsäureester mit Triacetin umgeestert.

### Stand der Technik

Bei der Verarbeitung, insbesondere der formgebenden Verarbeitung von halogenhaltigen organischen Kunststoffen, insbesondere Polyvinylchlorid (PVC), werden diesen Kunststoffen in der Regel Additive zugesetzt, die bestimmte Funktionen erfüllen sollen. Dabei kann es gewünscht sein, daß die Additive ihre Wirksamkeit während der Verarbeitung selbst entfalten oder daß sie den so zugänglichen Formkörpern bzw. Werkstücken bestimmte Eigenschaften verleihen.

Eine wichtige Klassse solcher Additive, die in breitem Maße eingesetzt werden, sind die sogenannten Weichmacher. Dabei dominieren - wie dem Fachmann bekannt - seit langem sogenannte Phthalate oder Adipate den Markt. Insbesondere sind Dioctylphthalat (DOP) und Dioctyladipat (DOA) Weichmacher, die in diesem Bereich standardmäßig zum Einsatz kommen.

Es besteht jedoch seit langem ein Bedürfnis, die genannten klassischen Weichmacher wie DOP oder DOA durch Weichmacher anderer Struktur zu ersetzen. Eine technische Lösung hierfür hat bereits die im Jahre 1959 publizierte **US-A-2 895 966** vorgeschlagen. Hier werden epoxidierte Monoglyceriddiacetate als Weichmacher für Kunststoffe vorgeschlagen, etwa Monoepoxystearyldiacetoglycerid. Es wird offenbart, daß derartige Verbindungen neben gewissen stabilisierenden Eigenschaften insbesondere weichmachende Wirkung haben und sich darüber hinaus gut in zahlreiche Kunststoffe einarbeiten lassen, d.h. mit diesen kompatibel sind.

Gemäß der genannten US-A-2 895 966 werden die Weichmacher hergestellt, indem zunächst Monoglyceriddiacetate auf Basis ungesättigter Fettsäuren hergestellt werden und diese anschließend epoxidiert werden. Der Einsatz der in US-A-2 895 966 vorgeschlagenen Weichmacher hat sich in der Praxis jedoch nicht durchgesetzt. Ein wesentlicher Grund hierfür mag darin zu sehen sein, daß die Herstellung dieser Weichmacher technisch aufwendig ist. Als konkrete technische Lehre offenbart die genannte US-A-2.895 966 nämlich, zunächst ein Öl mit einem entsprechenden Gehalt der Fettsäurebausteine an C=C-Doppelbindungen einer Umesterung mit Glycerin zu unterwerfen, dann eine Acetylierung durchzuführen und schließlich in dritter Stufe zu Epoxidieren. Im Anschluß an die einzelnen Verfahrensstufen erfolgt jeweils eine aufwendige Reinigung der erhaltenen Rohprodukte. Insbesondere: Nach der Umesterung mit Glycerin wird mit Wasser gewaschen, um gebildete Seife und Glycerin zu entfernen; nach der Acetylierung mit überschüssigem Acetanhydrid unter Schutzgas wird erneut mit Wasser gewaschen und anschließend getrocknet.

### Das gleiche mehrstufige Verfahren zur Herstellung von Monoglyceriddiacetaten - jedoch auf Basis von tierischen Fetten - und deren nachfolgende Epoxidierung wird in der US-A-3 049 504 offenbart

Im übrigen sei festgestellt, daß sich die technische Lehre der US-A-2 895 966 und der US-A-3 049 504 ausdrücklich auf Monoglyceriddiacetate richtet. Dies ist insbesondere dem die Spalten 1 und 2 überbrückenden Absatz in der US-A-2 895 966 zu entnehmen. Zwar wird einschränkend festgestellt, daß üblicherweisse komplexe Substanzmischungen vorliegen, die durchaus gewisse Anteile an Glyceriden mit nur einer Acetogruppe enthalten, jedoch wird unmittelbar und eindeutig offenbart, daß Monoglyceriddiacetate in diesen Gemischen die für die zu erzielende Wirkung und die Lösung der gestellten Aufgabe entscheidende und mengenmäßig dominierende Spezies sind.

### Beschreibung der Erfindung

Die **Aufgabe** der vorliegenden Erfindung bestand darin, ein verbessertes Verfahren zur Herstellung von Weichmachern für halogenhaltige Kunststoffe vom Typ der epoxidierten Glyceridacetate bereitzustellen und somit Wirkstoffmischungen an epoxidierten Glyceridacetaten bereitzustellen, die auf Grund ihrer spezifischen Zusammensetzung besonders als weichmachende Substanzen für PVC geeignet sind.

**Gegenstand** der vorliegenden Erfindung ist ein Verfahren zur Herstellung von epoxidierten Gyceridacetaten, wobei man Epoxyfettsäureester mit Triacetin umestert.

### Zum Begriff der "epoxidierten Glyceridacetate"

Das erfindungsgemäße Verfahren besteht darin, Epoxyfettsäureester mit Triacetin umzusetzen, eine Reaktion, die chemisch als Umesterung verstanden werden kann. Das nach dieser Umsetzung vorliegende Produkt ist nicht zwingend homogen im Sinne einer einzigen Molekülstruktur. Vielmehr wird dieses Produkt in aller Regel eine mehr oder minder komplexe Mischung unterschiedlicher chemischer Individuen darstellen. Gleichwohl wird auch eine solche Mischung im Rahmen der vorliegenden Erfindung aus pragmatischen Gründen als "epoxidiertes Glyceridacetat" bezeichnet. In Abhängigkeit vom gewünschten Umsetzungsverhältnis der beiden Reaktanden der Umesterung (siehe unten) können dabei epoxidierte Monoglyceriddiacetate oder epoxidierte Diglyceridmonoacetate die mengenmäßig dominierende Species der Produktmischung darstellen.

### Epoxyfettsäureester

Unter Epoxyfettsäureestern werden im Rahmen der vorliegenden Erfindung verstanden:
a) Monoester von Fettsäuren und 1-wertigen Alkoholen der Formel R¹-OH, worin der Rest R¹ einen Alkylrest mit 1 bis 18 C-Atomen bedeutet, der gesättigt oder ungesättigt, geradkettig oder verzweigt, aliphatisch oder cycloaliphatisch sein kann, mit der Maßgabe, daß der Fettsäurerest sich von einer Fettsäure mit 8 bis 24 C-Atomen ableitet, die mindestens eine C=C-Doppelbindung pro Molekül enthält und der zusätzlichen Maßgabe, daß mindestens eine C=C-Doppelbindung pro Fettsäurebaustein in epoxidierter Form vorliegt.
b) Triester von Fettsäuren und Glycerin, mit der Maßgabe, daß die Fettsäurereste sich von Fettsäuren mit 3 bis 24 C-Atomen ableiten, mit der Maßgabe, daß mindestens 30% der Fettsäurebausteine der Triester mindestens eine C=C-Doppelbindung enthalten und der zusätzlichen Maßgabe, daß mindestens eine C=C-Doppelbindung pro Molekül Triester in epoxidierter Form vorliegt.

Der Begriff "Fettsäure" ist dem Fachmann geläufig und kann beispielsweise dem Standardwerk **Römpps Chemie-Lexikon** entnommen werden (vergleiche 7. Auflage, Stuttgart 1973, Seiten 1107 - 1110).

Die Verbindungen b) stellen Triglyceride auf Basis der genannten Fettsäuren und Glycerin dar. Als solche Triglyceride können synthetisch hergestellte Verbindungen eingesetzt werden oder auch Fette und Öle natürlichen Ursprungs (zum Begriff "Fette und Öle" siehe etwa **Römpps Chemie-Lexikon,** vergleiche 7. Auflage, Stuttgart 1973, Seiten 1101-1106).

Vorzugsweise enthalten mindestens 50% - und insbesondere mindestens 80% - der Fettsäurebausteine der Triester b) mindestens eine C=C-Doppelbindung.

Die Maßgabe, daß mindestens eine C=C-Doppelbindung pro Molekül Triester b) in epoxidierter Form vorliegen muß, bezieht sich logischerweise nur auf diejenigen Moleküle des Triesters, bei denen mindestens eine C=C-Doppelbindung vorhanden ist. Denn ein Triester b) enthält zwar definitionsgemäß - insbesondere wenn er sich von Fetten und Ölen natürlichen Ursprungs ableitet - einen bestimmten Anteil an Fettsäurebausteinen mit mindestens einer C=C-Doppelbindung, aufgrund der statistischen Verteilung können aber im Triester auch Moleküle enthalten sein, deren Fettsäurebausteine alle gesättigt sind.

### Triacetin

Triacetin ist durch folgende Struktur charakterisiert:

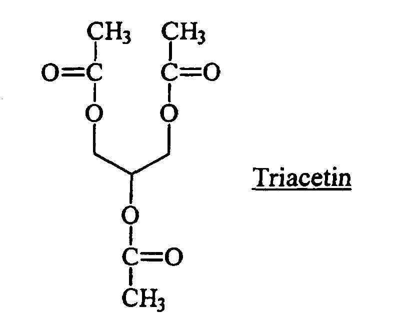

Triacetin ist ein kommerziell verfügbares Produkt, dessen Herstellung seit langem bekannt ist. So beschreibt beispielsweise bereits **DE-A-30 04 660** ein Verfahren zur Herstellung von Triacetin durch Umsetzung von Glycerin mit Essigsäure und Essigsäureanhydrid.

### Zur Umesterung von Epoxyfettsäureestern mit Triacetin

Das erfindungsgemäße Verfahren stellt eine Umesterung dar. Bei dieser Reaktion bleiben die Oxiranringe (= Epoxidgruppen) der Epoxyfettsäureester erhalten.

Das erfindungsgemäße Verfahren wird vorzugsweise in Gegenwart eines **Umesterungskatalysators** durchgeführt. Derartige Umesterungskatalysatoren sind dem Fachmann bekannt. Vorzugsweise kommen hier basische Verbindungen wie Natriumhydroxid, Kaliumhydroxid, Lithiumhydroxid, Natriummethylat, Natriumethylat in Betracht, ferner Zinn (IV)-Verbindungen, wie beispielsweise Dibutyl-zinn-di-laurat. Die eingesetzten Katalysatormengen liegen vorzugsweisse zwischen 0,01 und 1,0 Gew.-% - bezogen auf die Gesamtmenge an eingesetztem Epoxyfettsäureester und Triacetin - und insbesondere im Bereich von 0,05 bis 0,2 Gew.-%.

Das erfindungsgemäße Verfahren wird vorzugsweise bei **Reaktionstemperaturen** im Bereich von 120 und 240 °C, und insbesondere 150 bis 230 °C durchgeführt.

Die **Reaktionszeiten** betragen bei dem erfindungsgemäßen Verfahren vorzugsweise 1 bis 6 Stunden, und insbesondere 3 bis 5 Stunden.

Die molaren Verhältnisse von Epoxyfettsäureestern und Triacetin stellt man im erfindungsgemäßen Verfahren vorzugsweise auf Werte im Bereich von 1 : 2 bis 2 : 1 ein.

Als Epoxyfettsäureester werden im Rahmen des erfindungsgemäßen Verfahrens insbesondere eingesetzt:
- Epoxidierte Ester von Fettsäuren und 1-wertigen Alkoholen vom oben näher bezeichneten Typ a), wobei die Alkoholbausteine dieser Ester ausgewählt werden aus der Gruppe Methanol, Ethanol, Propanol, Butanol und 2-Ethylhexanol.
- Sojaöl-Epoxid, Leinöl-Epoxid, Sonnenblumenöl-Epoxid, Rüböl-Epoxid, Talg-Epoxid.

Das nach dem erfindungsgemäßen Verfahren erzeugte Reaktionsprodukt ist ein spezifisches Gemisch **epoxidierter Glyceridacetate**, das dadurch erhältlich ist, daß man Epoxyfettsäureester mit Triacetin umestert. Die molaren Verhältnisse von Epoxyfettsäureestern und Triacetin stellt man hierzu bei der Umesterung insbesondere auf Werte im Bereich von 1,1 : 1 bis 2 : 1 ein.

Diese Produktmischung findet Verwendung als Weichmacher für halogenhaltige organische Kunststoffe, insbesondere PVC.

### Beispiele

| Eingesetzte Substanzen | |
|---|---|
| **Bezeichnung** | **Erläuterung** |
| Vinnolit H 70 DF | PVC (Firma Advent International) |
| Irgastab BZ 561 | Ba/Zn-Stabilisator (Fa. Ciba-Geigy) |
| Edenol D 81 | epoxidiertes Sojaöl (Fa. Cognis / DE) |
| Stabiol CZ 2222 | Ca/Zn-Stabilisator (Fa. Cognis / DE) |
| Loxiol G 10 | Gleitmittel (Fa. Cognis / DE) |
| Loxiol G 20 | Gleitmittel (Fa. Cognis / DE) |
| Di-octyl-phthalat | Weichmacher (Fa. Degussa-Hüls / DE) |
| Di-octyl-adipat | Weichmacher (Fa. Degussa-Hüls / DE) |

### Herstellbeispiele

### Beispiel 1:

### Umesterung von Ölsäuremethylester-Epoxid mit Triacetin

145 g Triacetin wurden mit 205 g Ölsäuremethylester-Epoxid (Epoxid-Gehalt = 4,9 %) unter Einsatz von 1,8 g Natriummethylat als Katalysator bei 150 °C umgeestert. Die Reaktionsdauer betrug 3 Stunden. Es wurden ca. 30 g Essigsäuremethylester als Nebenprodukt abdestilliert. Es wurden 320 g Glycerin-monoepoxyoleat-di-acetat erhalten Bei diesem Produkt wurden folgende Kennzahlen gemessen:
Säurezahl (SZ): 0,97
Verseifungszahl (VZ): 390
Epoxid-Gehalt.: 3,1 %

Zur Verbesserung der Produktfarbe kann gewünschtenfalls mit ca. 2 g Wasserstoffperoxid (30 %ig) gebleicht werden.

### Beispiel 2:

### Umesterung von epoxidiertem Söjaöl mit Triacetin

340 g epoxidiertes Söjaöl (Edenol D 81) wurden mit 161 g Triacetin unter Einsatz von 0,25 g Natriumhydroxyd als Katalysator bei 220 °C umgeestert. Die Reaktionsdauer betru 4 Stunden. Zur Verbesserung der Produktfarbe wurde mit ca. 2 g Wasserstoffperoxid (30 %ig) gebleicht. Man erhielt ca. 500 g Glycerin-monoepoxyoleat-di-acetat. Bei diesem Produkt wurden folgende Kennzahlen gemessen:
SZ: 0,86
Lovibondfarbe 1": gelb: 5,6; rot: 1,0
Epoxid-Gehalt.: 4,1 %

### Beispiel 3:

### Umesterung von epox. Söjaöl mit Triacetin

340 g epoxidiertes Söjaöl (Edenol D 81) wurden mit 161 g Triacetin unter Einsatz von 0,25 g Dibutyl-zinn-di-laurat als Katalysator bei 220 °C umgeestert. Die Reaktionsdauer betrug 3 Stunden. Zur Verbesserung der Produktfarbe wurde mit ca. 1 g Wasserstoffperoxid (30 %ig) gebleicht. Man erhielt ca. 500 g Glycerin-monoepoxyoleat-di-acetat. Bei diesem Produkt wurden folgende Kennzahlen gemessen:
SZ: 1,2
Lovibondfarbe 1": gelb: 6,2; rot: 1,0
Epoxid-Gehalt: 4,0 %

### Anwendungsbeispiele

Zur anwendungstechnischen Prüfung wurden Rezepturen R1 bis R8 auf Basis verschiedener Weichmacher hergestellt. Die Zusammensetzung dieser Rezepturen, ist im folgenden tabellarisch wiedergegeben.

Die Mengenanteile der einzelnen Komponenten sind in phr angegeben; phr bedeutet dabei "part per hundred resin" und gibt an, wieviele Gewichtsteile der jeweiligen Substanz nach der Zugabe der Zusammensetzung im PVC - bezogen auf 100 Gewichtsteile PVC - vorhanden sind. Dementsprechend enthalten die Rezepturen jeweils 100 Teile PVC (Vinnolit H 70 DF).

Es sei ausdrücklich darauf hingewiesen, daß bei R1 bis R8 zwischen den Gesamtrezepturen, die zur anwendungstechnischen Prüfung herangezogen werden und die alle aufgeführten Komponenten enthalten und den eigentlichen Additiv-Zusammensetzungen, die alle aufgeführten Komponenten mit Ausnahme des PVC enthalten, zu unterscheiden ist.

| | R1 | R2 | **R3** |
|---|---|---|---|
| Vinnolit H 70 DF | 100 | 100 | **100** |
| Irgastab BZ 561 | 1,5 | 1,5 | **1,5** |
| Di-octyl-phthalat | 100 | 0 | **0** |
| Di-octyl-adipat | 0 | 100 | **0** |
| Verbindung nach Beispiel 1 | 0 | 0 | **100** |

| | R4 | **R5** | **R6** |
|---|---|---|---|
| Vinnolit H 70 DF | 100 | **100** | **100** |
| Edenol D 81 | 8 | **8** | **8** |
| Stabiol CZ 2222 | 1 | **1** | **1** |
| Loxiol G 10 | 2,5 | **2,5** | **2,5** |
| Loxiol G 20 | 0,2 | **0,2** | **0,2** |
| Di-octyl-phthalat | 30 | **0** | **0** |
| Verbindung nach Beispiel 2 | 0 | **30** | **0** |
| Verbindung nach Beispiel 3 | 0 | **0** | **30** |

| | R7 | **R8** |
|---|---|---|
| Vinnolit H 70 DF | 100 | **100** |
| Edenol D 81 | 8 | **8** |
| Stabiol CZ 2222 | 1 | **1** |
| Loxiol G 10 | 2,5 | **2,5** |
| Loxiol G 20 | 0,2 | **0,2** |
| Di-octyl-adipat | 50 | **0** |
| Verbindung nach Beispiel 3 | 0 | **50** |

Auf Basis der Rezepturen R1 bis R8 wurden Walzfelle hergestellt und die statische Thermostabilität bei 180°C bestimmt. Die Herstellung der Walzfelle erfolgte, indem man jeweils die genannten Rezepturen auf einem Laborwalzwerk 5 Minuten lang bei 170 °C homogenisierte und plastifizierte. Aus den so hergestellten etwa 0,5 mm dicken Walzfellen wurden Teststücke (Prüfkörper) der Größe 17 x 17 mm herausgeschnitten.

Bei den Prüfrezepturen wurden jeweils folgende Messungen durchgeführt:
- **Stabilitätstest bei thermischer Belastung:** Entsprechend den Rezepturen wurden Walzfelle hergestellt und die statische Thermostabilität bei 180°C bestimmt. Die Herstellung der Walzfelle erfolgte, indem man die Komponenten der genannten Rezepturen auf einem Laborwalzwerk 5 Minuten lang bei 170 °C homogenisierte und plastifizierte. Aus den so hergestellten etwa 0,5 mm dicken Walzfellen wurden Teststücke (Prüfkörper) der Größe von 17 x 17 mm herausgeschnitten. Die Prüfkörper wurden bei 180 °C im Wärmeschrank auf Glasplatten auf rotierenden Horden plaziert und in 15-minütigen Abständen wieder entnommen, bis alle Proben "verbrannt" waren (d.h. Schwarzfärbung erreicht hatten)
- **Farbmessung am Walzfell**: Darüber hinaus wurde bei den Walzfellen zur weiteren Charakterisierung die dem Fachmann bekannte L*,a*,b*-Methode (vergleiche DIN 6174) herangezogen. Bestimmt wurde die Anfangsfarbe des Walzfells (Gelbwert b*). Bei den Messungen kam ein handelsübliches Gerät mit der Bezeichnung "Micro Color" (Firma Dr. Lange) zum Einsatz.
- **Shore-A-Härte:** Die dem Fachmann einschlägig bekannte Shore-A-Härte der Walzfelle wurden mit einem handelsüblichen Härteprüfgerät nach Shore (Fa. Zwick) gemäß DIN 53505 bestimmt.
- **Lichtdurchlässigkeit:** Die Lichtdurchlässigkeit von 4 mm starken Preßplatten auf Basis der genannten Rezepturen wurde bestimmt. Die Herstellung der Preßplatte erfolgte ausgehend von den Walzfellen. Die Walzfelle wurden mit einer Laborpresse (Fa. Collin) zwischen zwei Preßblechen mittels Abstandhaltern zu Platten der genannten Stärke gepreßt. Bei den durchgeführten Untersuchungen wurden 8 Walzfelle einer Stärke von jeweils 0,5 mm bei 170 °C und 250 bar zu einer Preßplatte einer Stärke von 4 mm gepreßt.
- **Verträglichkeit:** Unter Verträglichkeit ist die Kompatibilität der Additiv-Zusammensetzungen mit dem Kunststoff (PVC) zu verstehen. Die Verträglichkeit wurde jeweils nach Lagerung der Walzfelle bei Raumtemperatur bestimmt. Zur Prüfung der Verträglichkeit wurde eine Sichtkontrolle der Walzfelle durchgeführt. Dabei wurde beurteilt, ob Ausschwitzungen an der Oberfläche des Walzfells zu beobachten waren. Eine starke Ausschwitzung ist nach dieser Methode ein Indikator für starke Unverträglichkeit, das Ausbleiben einer Ausschwitzung ein Indikator für sehr gute Verträglichkeit.

Die mit den Prüfkörpern erhaltenen Ergebnisse sind nachfolgend tabellarisch zusammengestellt. Dabei ist C1 ein Prüfkörper auf Basis der Additiv-Zusammensetzung R1, ist C2 ein Prüfkörper auf Basis der Additiv-Zusammensetzung R2, usw.

| | C1 | C2 | C3 |
|---|---|---|---|
| Thermostabilität (180 °C) | 75 min. | 60 min. | > 270 min. |
| Shore-Härte A | 61 | 58 | 65 |
| Transparenz (Lichtdurchlässigkeit in %, Preßplatte 4 mm) | 83 | 76 | 83 |
| Veträglichkeit nach Herstellung | sehr gut, keine Ausschwitzung | sehr gut, keine Ausschwitzung | sehr gut, keine Ausschwitzung |
| Veträglichkeit nach 6 Monaten (Lagerung bei Raumtemperatur) | sehr gut, keine Ausschwitzung | sehr gut, keine Ausschwitzung | sehr gut, keine Ausschwitzung |
| Veträglichkeit nach 12 Monaten (Lagerung bei Raumtemperatur) | sehr gut, keine Ausschwitzung | sehr gut, keine Ausschwitzung | sehr gut, keine Ausschwitzung |

| | C4 | C5 | C6 |
|---|---|---|---|
| Thermostabilität (180 °C) | 90 min. | 210 min. | 210 min. |
| Shore-Härte A | 90 | 93 | 93 |
| Anfangsfarbe des Walzfells (Gelbwert b*) | 4,7 | 6,3 | 5,8 |
| Veträglichkeit nach Herstellung | sehr gut, keine Ausschwitzung | sehr gut, keine Ausschwitzung | sehr gut, keine Ausschwitzung |
| Veträglichkeit nach 18 Wochen (Lagerung bei Raumtemperatur) | sehr gut, keine Ausschwitzung | sehr gut, keine Ausschwitzung | sehr gut, keine Ausschwitzung |

| | C7 | C8 |
|---|---|---|
| Thermostabilität (180 °C) | 75 min. | 240 min. |
| Shore-Härte A | 73 | 80 |
| Anfangsfarbe des Walzfells (Gelbwert b^{*}) | 2,6 | 4,3 |
| Veträglichkeit nach Herstellung | sehr gut, keine Ausschwitzung | sehr gut, keine Ausschwitzung |
| Veträglichkeit nach 10 Wochen (Lagerung bei Raumtemperatur) | sehr gut, keine Ausschwitzung | sehr gut, keine Ausschwitzung |

## Patentansprüche

1. Verfahren zur Herstellung von epoxidierten Glyceridacetaten, wobei man Epoxyfettsäureester mit Triacetin umestert.

2. Verfahren nach Anspruch 1, wobei man als Epoxyfettsäureester Triester von Fettsäuren und Glycerin einsetzt, wobei die Fettsäurereste dieser Triester sich von Fettsäuren mit 3 bis 24 C-Atomen ableiten, mit der Maßgabe, daß mindestens 80% der Fettsäurebausteine der Triester mindestens eine C=C-Doppelbindung enthalaten und der zusätzlichen Maßgabe, daß mindestens eine C=C-Doppelbindung pro Molekül Triester in epoxidierter Form vorliegen.

3. Verfahren nach Anspruch 1 oder 2, wobei man die Umesterung in Gegenwart eines Umesterungskatalysators durchführt.

4. Verfahren nach Anspruch 3, wobei man den Katalysator in einer Menge einsetzt, die zwischen 0,01 und 1,0 Gew.-% - bezogen auf die Gesamtmenge an eingesetztem Epoxyfettsäureester und Triacetin - liegt.

5. Verfahren nach einem der Ansprüche 1 bis 4, wobei man die Reaktionstemperatur auf Werte im Bereich von 120 und 240 °C einstellt.

6. Verfahren nach einem der Ansprüche 1 bis 5, wobei man die Reaktionszeit auf Werte im Bereich von 1 bis 6 Stunden einstellt.

7. Verfahren nach einem der Ansprüche 1 bis 6, wobei man die molaren Verhältnisse von Epoxyfettsäureestern und Triacetin auf Werte im Bereich von 1 : 2 bis 2 : 1 einstellt.

## Claims

1. A process for the production of epoxidized glyceride acetates, **characterized in that** epoxyfatty acid esters are transesterified with triacetin.

2. A process as claimed in claim 1, **characterized in that** the epoxyfatty acid esters used are triesters of fatty acids and glycerol, the fatty acid components of these triesters deriving from C₃₋₂₄ fatty acids, with the proviso that at least 80% of the fatty acid units of the triesters contain at least one C=C double bond and with the additional proviso that at least one C=C double bond per molecule of triester is present in epoxidized form.

3. A process as claimed in claim 1 or 2, **characterized in that** the transesterification is carried out in the presence of a transesterification catalyst.

4. A process as claimed in claim 3, **characterized in that** the catalyst is used in a quantity of 0.01 to 1.0% by weight, based on the total quantity of epoxyfatty acid ester used and triacetin.

5. A process as claimed in any of claims 1 to 4, **characterized in that** the reaction temperature is adjusted to a value of 120 to 240°C.

6. A process as claimed in any of claims 1 to 5, **characterized in that** the reaction time is adjusted to a value of 1 to 6 hours.

7. A process as claimed in any of claims 1 to 6, **characterized in that** the molar ratios of epoxyfatty acid esters to triacetin are adjusted to values of 1:2 to 2:1.

## Revendications

1. Procédé de préparation d'acétates de glycérides époxydés, selon lequel
on transestérifie des esters d'acides gras époxydés avec de la triacétine.

2. Procédé selon la revendication 1,
selon lequel
on utilise comme esters d'acides gras époxydés des triesters d'acides gras et de glycérine, les radicaux acide gras de ces triesters dérivant d'acides gras comportant de 3 à 24 atomes de carbone, étant précisé qu'au moins 80 % des constituants acide gras des triesters contiennent au moins une double liaison C=C et étant en outre précisé qu'au moins une double liaison C=C par molécule de triester se présente sous forme époxydée.

3. Procédé selon la revendication 1 ou 2,
selon lequel
on conduit la transestérification en présence d'un catalyseur de transesterification.

4. Procédé selon la revendication 3,
selon lequel
on utilise le catalyseur en une quantité située entre 0,01 et 1,0 % en poids - par rapport à la quantité totale d'ester d'acide gras époxydé, et de triacétine utilisée - .

5. Procédé selon l'une des revendications 1 à 4,
selon lequel
on règle la température de la réaction à des valeurs situées dans un intervalle allant de 120 à 240°C.

6. Procédé selon l'une des revendications 1 à 5,
selon lequel
on règle le temps de réaction à des valeurs situées dans un intervalle allant de 1 à 6 heures.

7. Procédé selon l'une des revendications 1 à 6,
selon lequel
le rapport molaire d'ester d'acide gras époxydé et de triacétine est réglé à des valeurs situées dans un intervalle allant de 1 : 2 à 2 : 1.
